# EUROPÄISCHE PATENTSCHRIFT

(11) **EP 2 260 505 B1**
(45) Veröffentlichungstag und Bekanntmachung des Hinweises auf die Patenterteilung: **28.08.2013**
(21) Anmeldenummer: 09730579.1
(22) Anmeldetag: 13.03.2009
(51) Int. Cl.: H01J 61/42, C09K 11/59, C09K 11/80, A61N 5/06

(54) **NIEDERDRUCKGASENTLADUNGSLAMPE ZUR BEEINFLUSSUNG DES KÖRPEREIGENEN MELATONINHAUSHALTES**
LOW-PRESSURE GAS DISCHARGE LAMP FOR INFLUENCING THE NATURAL MELATONIN BALANCE
LAMPE À DÉCHARGE À BASSE PRESSION POUR INFLUENCER LA PRODUCTION DE MÉLATONINE PROPRE AU CORPS

(30) Priorität: 08.04.2008 DE 102008017606
(43) Veröffentlichungstag der Anmeldung: 15.12.2010
(73) Patentinhaber: Merck Patent GmbH, 64293 Darmstadt (DE); NARVA Lichtquellen GmbH & Co. KG, 09618 Brand-Erbisdorf (DE)
(72) Erfinder: TEWS, Walter, 17489 Greifswald (DE); ROTH, Gundula, 17498 Levenhagen (DE); MELTKE, Juergen, 09618 Brand-Erbisdorf (DE); WALTHER, Hans-Juergen, 09618 Brand-Erbisdorf (DE)
(86) Internationale Anmeldenummer: PCT/EP2009/001821
(87) Internationale Veröffentlichungsnummer: WO 2009/124634

(56) Entgegenhaltungen:
- EP-A- 0 173 859
- EP-A- 1 154 461
- US-A1- 2003 160 560

## Beschreibung

Die Erfindung betrifft eine Niederdruckgasentladungslampe zur Beeinflussung des körpereigenen Melatoninhaushaltes, die vorzugsweise für biologisch-medizinische Anwendungen geeignet ist. Mit der erfindungsgemäßen Lichtquelle soll der biologische circadiane Rhythmus durch Steuerung der Melatoninsekretion gezielt beeinflusst werden.

### Stand der Technik

Aus der Medizin ist bekannt, dass fast alle menschlichen Prozesse biologischen Wirkungsfunktionen unterliegen. Eine davon ist die circadiane Wirkungsfunktion, durch die die Melatoninsekretion beeinflusst wird. Melatonin ist ein Schlafhormon, das von den Pinealozyten in der Zwirbeldrüse (Epiphyse), einem Teil des Zwischenhirns, aus Serotonin produziert und ausgeschüttet wird und den Tag-Nacht-Rhythmus des menschlichen Körpers steuert. Chemisch gesehen handelt es sich um ein Alkaloid mit Tryptamin-Struktur.

Durch Morita, Tekeshi und Tokura: The influence of different wavelengths of light on human biological rhythms, in Appl Human Sci, 17 (3): 91-96, 1998, wird die Beziehung zwischen der Qualität des Lichtes und dem menschlichen biologischen Rhythmus beschrieben. Es wurde festgestellt, dass sich das Verhalten der Kerntemperatur und der Veränderung der Melatoninsekretion in Abhängigkeit von der Wellenlänge des Lichtes ändert. Während Licht mit langer Wellenlänge wie das Licht mit niedriger Farbtemperatur, das rote Licht, keine Auswirkungen auf den menschlichen biologischen Rhythmus hat, zeigte dagegen das grüne und blaue Licht, das mit einer mittelkurzen Wellenlänge und hoher Farbtemperatur größere Wirkungen. Aus der Beziehung zwischen der Anregung der Photorezeptoren im menschlichen Auge und der Hemmung der Kerntemperatur und des Melatonins wird geschlussfolgert, dass der Photorezeptor nach erfolgtem Empfang der Lichtnachricht den biologischen Rhythmus mittels der Aktivität der Melatoninsekretion bestimmt. Es wurde festgestellt, dass am Morgen eine höhere Lichtintensität als am Abend benötigt wird, um die Hemmung der Melatoninsekretion einzuleiten. Der Einfluss auf den menschlichen biologischen Rhythmus während des gesamten Tages erfolgt mit den Photorezeptoren der Augen, die die Signale zur Melatoninsekretion empfangen.

Das Lebens- und Arbeitsumfeld ist abends und während der Nacht durch Licht mit niedriger Farbtemperatur und morgens in der Früh bis zum Abend durch Licht mit hoher Farbtemperatur zu beleuchten.

Durch das Licht mit einer Wellenlänge zwischen 400 nm und 500 nm und einem Wellenlängenmaximum bei 450 nm kann die Melatoninsuppression verstärkt und Leistungsvermögen und Wohlbefinden des Individuums erhöht werden. Bei Wegfall von Licht bei Dunkelheit lässt die Melatoninsuppression nach und das ausgeschüttete Melatonin leitet die Schlafphase ein.

Nach Bekanntwerden der Zusammenhänge zwischen Melatoninsuppression und Wohlbefinden durch Beeinflussung mit Licht im blauen Wellenlängenbereich sind Niederdruckgasentladungslampen zur Allgemeinbeleuchtung entwickelt worden, die erhöhte Blauanteile besitzen. In der Allgemeinbeleuchtung wird häufig mit der Farbtemperatur zur Unterscheidung verschiedener Lichtfarben gearbeitet. Die Erhöhung der Blauanteile führt danach zwangsläufig zu Lampen mit höheren Farbtemperaturen.

In EP 1735405 A1 wird eine Niederdruckgasentladungslampe beschrieben, deren Leuchtstoffe sich aus einem im roten Wellenlängenbereich emittierenden, mit Europium dotierten Yttrium-Leuchtstoff, einem im grünen Wellenlängenbereich emittierenden, mit Cerium und Terbium dotierten Lanthanphosphat-Leuchtstoff und einem im blauen Wellenlängenbereich emittierenden mit Europium dotierten Barium-Magnesiumaluminat-Leuchtstoff, einem im blauen Wellenlängenbereich emittierenden mit Mangan und Europium dotierten Mangan-Strontium-Barium-Magnesiumaluminat-Leuchtstoff zusammensetzen. Die Niederdruckgasentladungslampe erfüllt alle Forderungen zur Allgemeinbeleuchtung und erreicht vorzugsweise eine Farbtemperatur von 8000 K. Weiterhin wird genannt, dass Lampen mit Farbtemperaturen bis 20000 K denkbar wären. Diese Entwicklungsrichtung wird unter dem Gesichtspunkt verfolgt, durch Erhöhung des Blauanteils in der Allgemeinbeleuchtung durch Niederdruckgasentladungslampen den circadianen Rhythmus zu beeinflussen, die Melatoninsuppression zu verstärken und damit das menschliche Wohlbefinden und dessen Leistungsvermögen zu steigern. Eine andere Anwendung von Licht zur Beeinflussung des circadianen Rhythmus wird in DE 10 2005 059 518 A1 beschrieben, um den Melatoninanteil in der Milch von Säugetieren zu erhöhen. Die Melatoninsekretion ist dem circadianen Rhythmus folgend auch bei Säugetieren in den Nachtstunden am größten. Da ein Melken der Tiere in der Dunkelheit schwierig ist, werden in der zitierten Erfindung zur Gewährleistung der Blaufreiheit als Lichtquelle Natriumdampflampen oder vorzugsweise rot emittierende LED-Lampen verwendet. Der Nachteil in der Verwendung derartiger schmalbandiger Lampen besteht im Fehlen einer guten Farbwiedergabe, die für Sehaufgaben bedeutungsvoll ist.

Durch US20050179392 A1 ist eine Niederdruckgasentladungslampe bekannt, die ebenfalls für die Beeinflussung der Melatoninsekretion vorgesehen ist. Sie besitzt ein Entladungsgefäß, das mit einer inerten Gasmischung und Quecksilber ausgestattet ist. Das Entladungsgefäß beinhaltet zwei symmetrisch zueinander angeordnete und miteinander verbundene Entladungsräume. Der erste und zweite Entladungsraum ist jeweils mit einer Elektrode und einer Leuchtschicht ausgerüstet. Der Leuchtstoff im ersten Entladungsraum strahlt Licht in einem ersten Bereich des elektromagnetischen Spektrums von 100 nm bis 1000 nm aus und der Leuchtstoff im zweiten Entladungsraum strahlt Licht in einem zweiten Bereich des elektromagnetischen Spektrums von 100 nm bis 1000 nm aus, wobei sich das Emissionsspektrum beider Leuchtstoffe unterscheidet. Die Niederdruckgasentladungslampe beinhaltet für jeden Entladungsraum zwei Stromleiter zur Aufnahme der Gleichstromversorgung. Die Lampe hat eine variable Farbtemperatur und beeinflusst den menschlichen biologischen Rhythmus. Der erzeugte Lichtstrompegel ist unabhängig von der Temperatur. Die Lampe wird vorzugsweise für die Schichtarbeit, in Erste Hilfe-Stationen in Krankenhäusern usw. eingesetzt.

In EP 1886708 A1 ist eine Leuchte mit Melatonin schonender Wirkung beschrieben worden. Die Leuchte wird mit drei Leuchtstofflampen angesteuert und kann Licht in unterschiedlichen spektralen Zusammensetzungen bzw. Farbtemperaturen erzeugen, wobei bei der Ansteuerung die spektrale Zusammensetzung des erzeugten Lichtes in Abhängigkeit von einem vorgegebenen Zeitschema frei wählbar ist. In einem vorab definierten Zeitraum, insbesondere nachts, wird dabei ein Melatonin-Licht erzeugt, das hinsichtlich seiner spektralen Zusammensetzung und Intensität derart ist, dass es bei den Individuen eine nächtliche Ausschüttung des Hormons Melatonin zumindest weitestgehend nicht hemmt. Mit Bezug auf die spektrale Zusammensetzung des Melatonin-Lichtes ist vorgesehen, dass der Anteil des blauen Spektrums unterhalb des Wellenlängen-Schwellenwertes zwischen 480 nm und 600 nm, vorzugsweise zwischen etwa 500 nm und 560 nm liegt oder dass das Melatonin-Licht eine Farbtemperatur aufweist, die kleiner als beispielsweise 1500 K ist. Auf diese Weise wird verhindert, dass bei nächtlichem Kunstlicht die natürliche Melatoninsekretion eines Individuums, das diesem Licht ausgesetzt ist, unerwünscht gehemmt wird.

Nach Christoph Schierz: Der Mensch im farbigen Licht, in Licht 2006: 17. Lichttechnische Gemeinschaftstagung der Schweizer Licht Gesellschaft und der lichttechnischen Gesellschaften Deutschlands, Österreichs und der Niederlande, 10. bis 13. Sept. 2006, Bern, zeigt sich die spektrale Empfindlichkeit der Melatoninsuppression gegenüber der spektralen Hellempfindlichkeitsfunktion V(λ) als in den blau erscheinenden Bereich des Spektrums verschoben. Es wird gezeigt, dass eine ähnliche spektrale Empfindlichkeit auch für die zeitliche Stabilisierung der biologischen Uhr und für die Steigerung des subjektiven und physiologischen Wachheitsgrades gelten muss. Die neuesten wissenschaftlichen Erkenntnisse zur biologischen Wirkung von Licht und Farbe lauten: Blau regt an und aktiviert, Rot hat diesbezüglich keine oder sogar eine hemmende Wirkung.

Der bekannte Stand der Technik lässt erkennen, dass die Erkenntnisse zum circadianen Rhythmus nur zur Entwicklung von Niederdruckgasentladungslampen mit höheren Farbtemperaturen und höheren Blauanteilen bei gleichzeitiger Sicherung der Sehaufgaben und Einhaltung gültiger Vorschriften zur Energieeffizienz geführt haben.

### Aufgabenstellung

Die Aufgabe der Erfindung besteht in einer Lichtquelle, mit der gezielt der circadiane Rhythmus durch Melatoninsekretion beeinflusst werden kann. In Verbindung mit den Lichtquellen zur Melatoninsuppression soll die Lichtquelle bei Fehlen äußerer Bedingungen wie Sonnenlicht besonders zur Simulierung eines quasicircadianen Rhythmus geeignet sein. Gleichzeitig soll die Niederdruckgasentladungslampe alle notwendigen Anforderungen an eine Lichtquelle für Beleuchtungsaufgaben durch gute Farbwiedergabe und hohe Energieeffizienz erfüllen und damit auch in der Allgemeinbeleuchtung einsetzbar sein.

Die erfindungsgemäße Niederdruckgasentladungslampe soll dabei die bisher gültigen Anforderungen für die Sehaufgaben, die an Lichtquellen dieser Art gestellt werden, erfüllen. Dazu gehören hoher Lichtstrom, gute Farbwiedergabe und die Erfüllung gültiger Vorschriften zur Energieeffizienz.

### Darlegung der Erfindung

Die Aufgabe wird gelöst durch die Herstellung einer Niederdruckgasentladungslampe, deren Blauanteile in der spektralen Energieverteilung im Wellenlängenbereich zwischen 400 nm und 500 nm äußerst gering sind und unterhalb des Schwellwertes für die Melatoninsuppression liegen.

Die erfindungsgemäßen Niederdruckgasentladungslampe zur Beeinflussung des körpereigenen Melatoninhaushaltes besteht aus dem bekannten Glasrohr. Das gläserne Entladungsgefäß kann erfindungsgemäß auch die Form einer Doppelhelix besitzen. Das Entladungsgefäß kann auch aus zusammen geschalteten U-Formen oder aus kurzen Rohrabschnitten, die über Verbindungskanälen zusammengefügt sind, bestehen.

Bei einer Form der Niederdruckgasentladungslampe wird das Entladungsrohr durch Faltung, Verformung, Verschweißung oder Fügen in eine Form gebracht, bei der die äußeren Abmessungen des Entladungsgefäßes nur einen Bruchteil der gestreckten Länge des Entladungsgefäßes betragen.

Das Entladungsgefäß der Niederdruckgasentladungslampe ist in bekannter Weise mit einem Edelgas bzw. Edelgasgemisch und Quecksilber gefüllt, und besitzt an beiden Enden Elektroden zur Einspeisung von Energie. Die Innenseite des Entladungsgefäßes ist mehrschichtig mit Leuchtstoff beschichtet.

Die erfindungsgemäße Niederdruckgasentladungslampe hat nacheinander auf die Glasoberfläche der Innenseite des Endladungsgefäßes aufgetragene Leuchtstoff- schichten. Die erste Leuchtstoffschicht, die als Grundschicht auf der Glasoberfläche aufgetragen ist, besteht aus einem Leuchtstoff, der angeregt wird sowohl durch die ultraviolette Quecksilberstrahlung zwischen 180 nm und 400 nm als auch durch die von der Quecksilberentladung emittierten blauen Strahlung zwischen 400 nm und 490 nm sowie durch die emittierte sichtbare Strahlung der zweiten oder weiteren Leuchtstoffschichten zwischen 400 nm und 550 nm angeregt wird, wobei sichtbares Licht mit Emissionsmaxima im Bereich zwischen 500 nm und 650 nm erzeugt wird und die zweite oder weiteren Leuchtstoffschichten, die als Deckschicht dienen, bestehen aus einem oder mehreren schmalbandig emittierenden Leuchtstoffen, die sichtbares Licht erzeugen.

Die unmittelbar auf der Glasoberfläche aufgetragene erste Leuchtstoffschicht besteht aus einem Leuchtstoff oder ein Leuchtstoffgemisch aus der Gruppe der Silikate oder Aluminate und ist vorzugsweise mit Europium oder Cerium dotiert. Dieser Leuchtstoff gehört der Gruppe der Erdalkali-Orthosilikate und/oder Erdalkali-Oxy-Orthosilikate, aktiviert mit zweiwertigem Europium, an und die Ionen können weitere Seltenerdmetalle sowie Mangan, Zink und Magnesium enthalten.

Der Leuchtstoff aus einem Eu-aktivierten Erdalkali-Orthosilikat hat die Formel (Ba, Sr, Ca)₂SiO₄:Eu und die Bezeichnung BOSE.

Der Leuchtstoff aus einem Eu-aktivierten Erdalkali-Oxy-Orthosilikat hat die Formel (Ba, Sr, Ca)₃SiO₅:Eu und die Bezeichnung SOOS.

Der Aluminat-Leuchtstoff aus der Gruppe der Granate hat die Formel Y₃Al₅O₁₂:Ce und die Bezeichnung YAG.

Der Leuchtstoff der ersten Leuchtstoffschicht auf der Innenseite des Entladungsgefäßes der Niederdruckgasentladungslampe weist ein Emissionsmaximum im Spektralbereich zwischen 560 nm und 650 nm auf. Die zweite Leuchtstoffschicht besteht aus einem oder mehreren schmalbandig emittierenden Leuchtstoffen, die bei einem Emissionsmaximum zwischen 440 nm und 500 nm in blau/türkis und/oder bei einem Emissionsmaximum zwischen 535 nm und 555 nm in grün und/oder bei einem Emissionsmaximum zwischen 605 nm und 650 nm in rot emittieren. Der rot emittierende Leuchtstoff ist vorzugsweise ein Yttriumoxid:Eu und/oder ein Yttriumvanadat:Eu und/oder ein Magnesiumfluorogermanat:Mn. Der grün emittierende Leuchtstoff ist ein Lanthanphosphat:Ce,Tb und/oder ein Magnesiumaluminat:Ce,Tb. Der blau/türkis emittierende Leuchtstoff ist ein Barium-Magnesiumaluminat:Eu und/oder ein Strontium-Calcium-Barium-Phosphat:Eu und/oder ein Strontiumaluminat:Eu und/oder ein Strontiumchlorphosphat:Eu.

Der Anteil des blau emittierenden Leuchtstoffes beträgt im Gemisch zwischen 0% und 10%.

Mit der Erfindung ist es möglich, in Kombination mit den bereits bestehenden Niederdruckgasentladungslampen mit hoher Farbtemperatur und entsprechend hohem Blauanteil, die zur Melatoninsuppression führen, die Schlafphase zu beeinflussen und somit den gesamten circadianen Rhythmus des Menschen zu stimulieren.

In Niederdruckgasentladungslampen herkömmlicher Ausführung resultieren die Blauanteile im sichtbaren Spektrum aus zwei Emissionsquellen unterschiedlicher Art: dem Emissionsspektrum von Leuchtstoffen und aus der Emission der Quecksilberlinien der Niederdruckgasentladung selbst bei 405 nm und 436 nm. Die Emissionslinie bei 436 nm ist mit 47,5% die stärkste in der Energieverteilung der Queckslberlinien im sichtbaren Spektrum und kann in bekannter Weise in jeder Niederdruckgasentladungslampe nachgewiesen werden.

Zur Melatoninsekretion mit Niederdruckgasentladungslampen ist auf Grund der Quecksilberemissionslinie bei 436 nm der Verzicht auf Leuchtstoffe mit einer Emission im Bereich von 400 nm bis 500 nm nicht ausreichend.

Der Zweck der erfindungsgemäßen Niederdruckgasentladungslampe zur Melatoninsekretion ist nur durch Absorption der Quecksilberemissionslinie bei 405 nm sowie vor allem bei 436 nm zu erreichen und wird mit einem blau absorbierenden Leuchtstoff ausgeführt.

Der blau absorbierende Leuchtstoff hat ein Anregungsspektrum zwischen 180 nm und 550 nm und erzeugt sichtbares Licht mit einem Emissionsmaximum im Bereich zwischen 500 nm und 650 nm.

Die spektrale Verteilung und die Stabilität des blau absorbierenden Leuchtstoffs sind jedoch nicht ausreichend, um die Forderung nach einer Niederdruckgasentladungslampe mit guter Sehwertfunktion zu erfüllen. Daher wird die erste Leuchtstoffschicht, die aus dem blau absorbierenden Leuchtstoff besteht, mit einer zweiten Leuchtstoffschicht aus der Mischung eines rot und grün emittierenden schmalbandigen Leuchtstoffs überzogen. Durch das Fehlen der Blauanteile in der spektralen Verteilung des sichtbaren Spektrums beträgt die Farbtemperatur nur 1000 K bis 3000 K, vorzugsweise 2000 K.

Durch Optimierung des Emissionsmaximums des blau absorbierenden Leuchtstoffs in der Grundschicht, der Zusammensetzung in der Leuchtstoffmischung der Deckschicht und der Schichtdicke beider Schichten erhält die erfindungsgemäße Niederdruckgasentladungslampe die Eigenschaften, die zur Erfüllung der Sehaufgaben benötigt werden.

### Ausführungsbeispiel

Die erfindungsgemäße Niederdruckgasentladungslampe soll anhand eines Ausführungsbeispiels näher erläutert werden.

In Tab. 1 sind die lichttechnischen Parameter der zwei erfindungsgemäßen Niederdruckgasentladungslampen LT 18W im Vergleich mit zwei herkömmlichen Niederdruckgasentladungslampen LT 18W dargestellt.

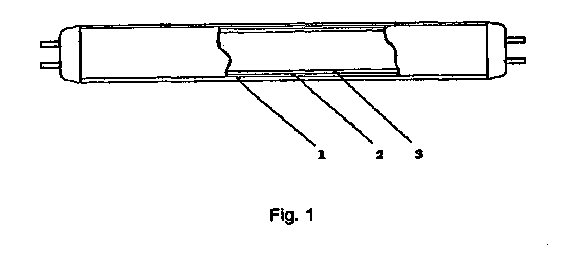

Die erfindungsgemäße Niederdruckgasentladungslampe zur Beeinflussung des körpereigenen Melatoninhaushaltes besitzt gemäß Fig. 1 das gläserne Entladungsgefäß 1 mit der Form eines geraden Rohres, das an beiden Enden Elektroden zur Einspeisung von Energie besitzt und mit einem Edelgas bzw. Edelgasgemisch und Quecksilber gefüllt ist. Auf der inneren Glasoberfläche des Entladungsgefäß sind nacheinander die Leuchtstoffschicht 2 als Grundschicht und auf dieser die Leuchtstoffschicht 3 als Deckschicht aufgebracht.

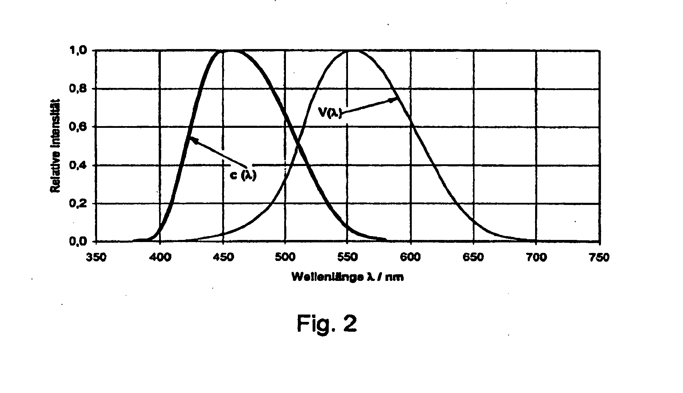

Die Fig. 2 zeigt die circadiane Wirkungsfunktion c(λ) und die spektrale Hellempfindlichkeitskurve V(λ) der erfindungsgemäßen Niederdruckgasentladungslampe LT 18W.

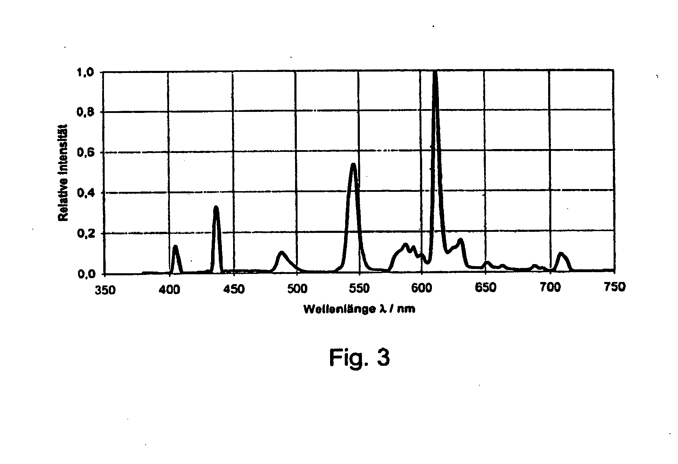

Das Spektrum der bekannten Drei-Banden-Niederdruckgasentladungslampe LT 18W mit der Lichtfarbe warmwhite-comfort ist in der Fig. 3 aufgezeichnet.

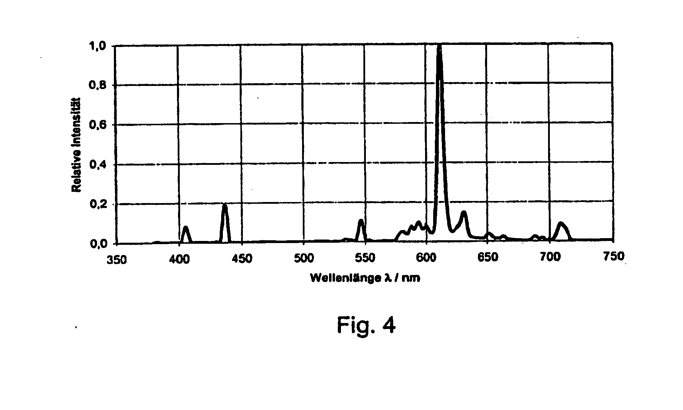

Das Spektrum der bekannten Niederdruckgasentladungslampe LT 18W mit Lichtfarbe rot ist in der Fig. 4 dargestellt.

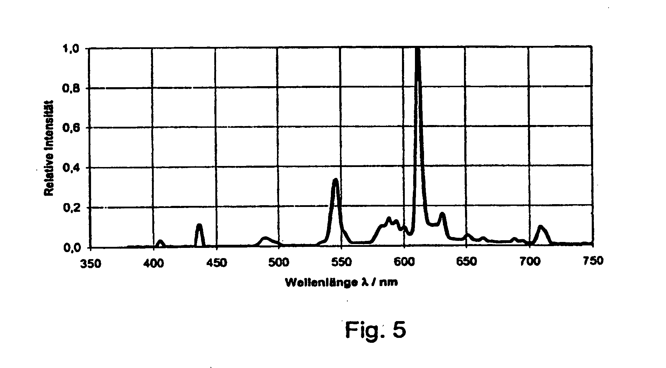

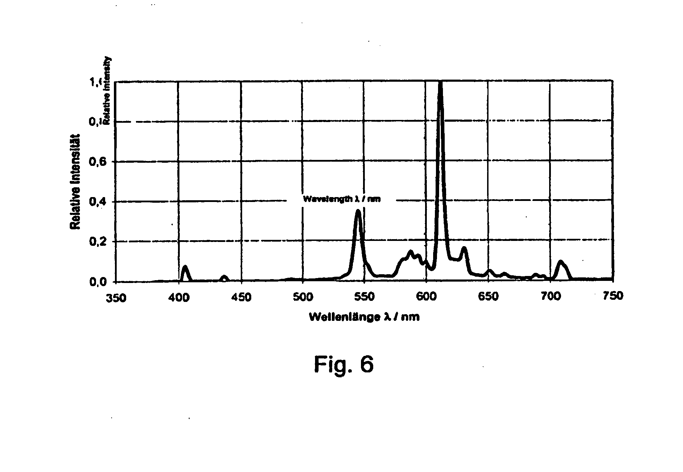

Zum Vergleich dazu zeigen die Fig. 5 das Spektrum der erfindungsgemäßen Niederdruckgasentladungslampe LT 18W mit dem als Grundschicht dienenden Leuchtstoff (Ba, Sr, Ca)Orthosilikat:Eu, der mit BSCOSE (F612) bezeichnet wird, und die Fig. 6 das Spektrum der erfindungsgemäßen Niederdruckgasentladunglampe LT 18W mit dem Leuchtstoff YAG als Grundschicht.

In beiden Ausführungsbeispielen werden Niederdruckgasentladunglampe LT 18W mit der Standardlänge von 590 mm und Glasrohrdurchmesser von 26 mm bei einer Lampenleistung von 18 W beschrieben.

### Beispiel 1

In dem Entladungsgefäß der Niederdruckgasentladunglampe LT 18W gelangt als blau absorbierender Leuchtstoff ein Eu-aktiviertes Erdalkali-Orthosilikat zur Anwendung.

Die Herstellung der Grundschicht erfolgt durch Beschichtung des blau absorbierenden Leuchtstoffs auf der Innenseite des Glaskolbens als Suspension, mit Hydroxyethylcellulose als Bindemittel und Dimethylolharnstoff als Vernetzer. Die Schichtdicke der Grundschicht liegt erfindungsgemäß zwischen 0,2 mg/cm² und 2,0 mg/cm², vorzugsweise bei 1,25 mg/cm²_{.}

Als Beschichtung für die zweite Schicht als Deckschicht wird eine Leuchtstoffmischung aus dem schmalbandig rot emittierenden Leuchtstoff Yttriumoxid:Eu (YOX) und dem schmalbandig grün emittierenden Leuchtstoff Lanthanphosphat:Ce,Tb (LAP) als Suspension mit Polyethylenoxid als Bindemittel verwendet. Die Leuchtstoffmischung besteht zu 60% bis 99% aus dem Leuchtstoff YOX und zu 1 % bis 40% aus dem Leuchtstoffes LAP, vorzugsweise zu 85% aus dem Leuchtstoff YOX und zu 15% aus dem Leuchtstoffes LAP.

Die Schichtdicke der Deckschicht ist erfindungsgemäß bemessen zwischen 1,0 mg/cm² und 6,0 mg/cm², vorzugsweise bei 3,25 mg/cm².

Der beschichtete Glaskolben wird ausgebrannt und danach der herkömmlichen Lampenfertigungstechnologie von Niederdruckgasentladungslampen unterzogen.

Die Niederdruckgasentladungslampe LT 18W, die mit dem BSCOSE-Leuchtstoff F612 als Grundschicht hergestellt ist, weist folgende lichttechnische Parameter auf:

| | |
|---|---|
| Lichtstrom: | 1341 Im |
| Lampenleistung: | 18,0 W |
| Farbtemperatur: | 1995 K |
| Farbwiedergabe: | 81,3. |

### Beispiel 2

Die Beschichtung des Glaskolbens der Niederdruckgasentladungslampe LT 18W wird mit dem Leuchtstoff YAG als Grundschicht durchgeführt. Die Beschichtung der Deckschicht erfolgt analog dem Beispiel 1.

Mit der so hergestellten Niederdruckgasentladungslampe LT 18W werden folgende lichttechnische Parameter erreicht:

| | |
|---|---|
| Lichtstrom: | 1408 Im |
| Lampenleistung: | 18,0 W |
| Farbtemperatur: | 1995 K |
| Farbwiedergabe: | 81,3. |

Der Lichtstrom wird entsprechend der spektralen Hellempfindlichkeitskurve V(A), wie in Fig. 2 dargestellt, mit einem Wellenlängenmaximum von 550 nm bewertet.

Zur Charakterisierung der Melatoninsekretion der erfindungsgemäßen Niederdruckgasentladungslampe wird der circadiane Wirkungsfaktor verwendet, der sich aus dem Verhältnis der integralen Produkte von Bestrahlungsstärke und circadianer Wirkungsfunktion c(A) und Bestrahlungsstärke und spektraler Hellempfindlichkeitskurve V(A) berechnet wird.

In Tab. 1 ist der circadiane Wirkungsfaktor der erfindungsgemäßen Niederdruckgas- entladurlgslampen LT 18W aus den Beispielen 1 und 2 im Vergleich zu herkömmlichen Niederdruckgasentladungslampen gleicher Lampenleistung in den Lichtfarben warmwhite-comfort und rot dargestellt. Gleichzeitig werden in Tab. 1 die lichttechnischen Parameter der vier Niederdruckgasentladungslampen LT 18W miteinander verglichen.

**Tab. 1**

| Niederdruckgasentladungslampe LT 18W mit: | Circadianer Wirkungsfaktor | Farbtemperatur | Lichtstrom | Farbwiedergabeindex |
|---|---|---|---|---|
| Lichtfarbe warmwhite-comfort | 0,301 | 2642 K | 1344 Im | 83,7 |
| Lichtfarbe rot | 0,178 | 1250 K | 955 Im | 49,2 |
| BSCOSE als Grundschicht/ Beispiel 1 | 0,146 | 1995 K | 1341 Im | 81,3 |
| mit YAG als Grundschicht/ Beispiel 2 | 0,071 | 1945 K | 1408 Im | 81,3 |

Die Niederdruckgasentladungslampe LT 18W in der Lichtfarbe warmwhite-comfort besitzt den höchsten circadianen Wirkungsfaktor der vier Niederdruckgas- entladungslampen. Das Spektrum dieser Niederdruckgasentladungslampe, wie in Fig. 3 dargestellt, enthält außer den Quecksilberlinien noch Blauanteile aus der Leuchtstoffemission, die damit im Gegensatz zu den anderen drei Niederdruckgasentladungslampen den höchsten circadianen Faktor hervorrufen.

Die Niederdruckgasentladungslampe LT 18W in der Lichtfarbe rot, in Fig. 4 dargestellt, enthält leuchtstoffseitig keine Blauanteile aus einer Leuchtstoffemission. Die Blauanteile dieser Niederdruckgasentladungslampe werden allein durch die Emission der sichtbaren Linien aus der Quecksilberentladung verursacht. Diese Niederdruckgasentladungslampe besitzt dadurch einen geringeren circadianen Faktor als die Niederdruckgasentladungslampe der Lichtfarbe warmwhite-comfort, ist aber auf Grund des sehr niedrigen Farbwiedergabeindexes für Sehaufgaben ungeeignet.

In den erfindungsgemäßen Niederdruckgasentladungslampen der Beispiele 1 und 2, deren Spektren in Fig. 5 und Fig. 6 dargestellt sind, erfolgt eine Anregung des Leuchtstoffs der Grundschicht durch die Quecksilberlinien bei 405 nm und 436 nm und einer Emission im sichtbaren Bereich von gelb bis rotorange.

Die erfindungsgemäßen Niederdruckgasentladungslampen der Beispiele 1 und 2 sind für Sehaufgaben gut geeignet. Die Niederdruckgasentladungslampe im Beispiel 1, die den Leuchtstoff BSCOSE (F612) in der Grundschicht hat, besitzt wegen der größeren Nähe zur Vergleichslichtart am Planckschen Strahler eine bessere Sehfunktion als die Niederdruckgasentladungslampe des Beispiels 2 mit dem Leuchtstoff YAG, die aber im circadianen Wirkungsfaktor geringer ist und damit die Melatoninsekretion steigert.

In Verbindung mit Lichtquellen zur Melatoninsuppression ist die erfindungsgemäße Niederdruckgasentladungslampe mit ihrem warmen Licht deshalb bei Fehlen äußerer Bedingungen wie Sonnenlicht besonders zur Simulierung des quasicircadianen Rhythmus beim Individuum geeignet. Gleichzeitig erfüllt die neue Niederdruck- gasentladungslampe alle notwendigen Anforderungen an eine Lichtquelle für Beleuchtungsaufgaben durch gute Farbwiedergabe und eine hohe Energieeffizienz und ist damit auch zur Allgemeinbeleuchtung geeignet.

## Patentansprüche

1. Niederdruckgasentladungslampe zur Beeinflussung des körpereigenen Melatoninhaushaltes, bestehend aus einem Entladungsrohr aus Glas, das innenseitig mehrschichtig mit Leuchtstoff beschichtet ist und an beiden Enden des Entladungsrohres Elektroden zur Einspeisung von Energie besitzt und mit einem Edelgas bzw. Edelgasgemisch und Quecksilber gefüllt ist, **dadurch gekennzeichnet, dass** die Leuchtstoffschichten nacheinander aufgetragen sind, wobei die auf der Glasoberfläche (1) befindliche erste Leuchtstoffschicht (2) aus einem Eu-aktivierten Erdalkaliorthosilikat mit der Formel (Ba,Sr,Ca)₂SiO₄:Eu (BOSE) oder einem Eu-aktivierten Erdalkali-Oxy-Orthosilikat mit der Formel (Ba,Sr,Ca)₃SiO₅:Eu (SOOS) oder einem Aluminat aus der Gruppe der Granate mit der Formel Y₃Al₅O₁₂:Ce (YAG) besteht, das sowohl durch die ultraviolette Quecksilberstrahlung zwischen 180 nm und 400 nm als auch durch die von der Quecksilberentladung sowie der zweiten oder weiteren Leuchtstoffschichten (3) emittierte sichtbare Strahlung zwischen 400 nm und 550 nm angeregt wird, wobei sichtbares Licht mit Emissionsmaxima im Bereich zwischen 500 nm und 650 nm erzeugt wird.

2. Niederdruckgasentladungslampe nach Anspruch 1, **dadurch gekennzeichnet, dass** die Schichtdicke der ersten Leuchtstoffschicht, die als Grundschicht dient, zwischen 0,2 mg/cm² und 2,0 mg/cm², vorzugsweise bei 1,25 mg/cm² und die Schichtdicke der zweiten Leuchtstoffschicht, die als Deckschicht dient, zwischen 1,0 mg/cm² und 6,0 mg/cm², vorzugsweise bei 3,25 mg/cm² bemessen ist.

3. Niederdruckgasentladungslampe nach Anspruch 1 und/oder 2, **dadurch gekennzeichnet, dass** der Leuchtstoff der ersten Leuchtstoffschicht ein Emissionsmaximum im Spektralbereich zwischen 560 nm und 620 nm aufweist.

4. Niederdruckgasentladungslampe nach einem oder mehreren der Ansprüche 1 bis 3, **dadurch gekennzeichnet, dass** die zweite Leuchtstoffschicht aus einem oder mehreren schmalbandig emittierenden Leuchtstoffen besteht, die bei einem Emissionsmaximum zwischen 440 nm und 500 nm in blau/türkis und/oder bei einem Emissionsmaximum zwischen 535 nm bis 555 nm in grün und/oder bei einem Emissionsmaximum zwischen 605 nm bis 630 nm in rot strahlen.

5. Niederdruckgasentladungslampe nach Anspruch 4, **dadurch gekennzeichnet, dass** der rot emittierende Leuchtstoff ein Yttriumoxid:Eu und/oder ein Yttriumvanadat:Eu und/oder ein Magnesiumfluorogermanat:Mn, der grün emittierende Leuchtstoff ein Lanthanphosphat:Ce, Tb und/oder ein Magnesiumaluminat:Ce, Tb und der blau/türkis emittierende Leuchtstoff ein Barium-Magnesiumaluminat:Eu und/oder ein Strontium-Calcium-Bariumphosphat:Eu und/oder ein Strontiumaluminat:Eu und/oder ein Strontiumchlorphosphat:Eu ist.

6. Niederdruckgasentladungslampe nach Anspruch 4 und/oder 5, **dadurch gekennzeichnet, dass** der Anteil des blau emittierenden Leuchtstoffes im Gemisch zwischen 0% und 10% beträgt.

7. Niederdruckgasentladungslampe nach Anspruch 1, **dadurch gekennzeichnet, dass** das Entladungsrohr durch Faltung, Verformung, Verschweißung oder Fügen in eine Form gebracht wird, bei der die äußeren Abmessungen des Entladungsgefäßes nur einen Bruchteil der gestreckten Länge des Entladungsgefäßes betragen.

8. Niederdruckgasentladungslampe nach Anspruch 1 und/oder 7, **dadurch gekennzeichnet, dass** das Entladungsgefäß als eine Doppelhelix ausgeformt ist oder aus zusammen geschalteten U-Formen und aus über Verbindungskanälen zusammengefügten kurzen Rohrab-schnitten gefertigt ist.

## Claims

1. Low-pressure gas-discharge lamp for influencing the endogenous melatonin balance, consisting of a discharge tube made from glass which is multicoated with phosphor on the inside and has at both ends of the discharge tube electrodes for the supply of energy and is filled with a noble gas or noble-gas mixture and mercury, **characterised in that** the phosphor layers are applied one after the other, where the first phosphor layer (2) located on the glass surface (1) consists of an Eu-activated alkaline-earth metal orthosilicate having the formula (Ba,Sr,Ca)₂SiO₄:Eu (BOSE) or an Eu-activated alkaline-earth metal oxyorthosilicate having the formula (Ba,Sr,Ca)₃SiO₅:Eu (SOOS) or an aluminate from the group of the garnets having the formula Y₃Al₅O₁₂:Ce (YAG), which is excited both by the ultraviolet mercury radiation between 180 nm and 400 nm and also by the visible radiation between 400 nm and 550 nm emitted by the mercury discharge and the second or further phosphor layers (3), where visible light having emission maxima in the range between 500 nm and 650 nm is generated.

2. Low-pressure gas-discharge lamp according to Claim 1, **characterised in that** the layer thickness of the first phosphor layer which serves as base layer is set between 0.2 mg/cm² and 2.0 mg/cm², preferably 1.25 mg/cm², and the layer thickness of the second phosphor layer which serves as top layer is set between 1.0 mg/cm² and 6.0 mg/cm², preferably 3.25 mg/cm².

3. Low-pressure gas-discharge lamp according to Claim 1 and/or 2, **characterised in that** the phosphor in the first phosphor layer has an emission maximum in the spectral region between 560 nm and 620 nm.

4. Low-pressure gas-discharge lamp according to one or more of Claims 1 to 3, **characterised in that** the second phosphor layer consists of one or more narrowband-emitting phosphors which emit in blue/turquoise at an emission maximum between 440 nm and 500 nm and/or in green at an emission maximum between 535 nm and 555 nm and/or in red at an emission maximum between 605 nm and 630 nm.

5. Low-pressure gas-discharge lamp according to Claim 4, **characterised in that** the red-emitting phosphor is an yttrium oxide:Eu and/or yttrium vanadate:Eu and/or magnesium fluorogermanate:Mn, the green-emitting phosphor is a lanthanum phosphate:Ce,Tb and/or magnesium aluminate:Ce,Tb, and the blue/turquoise-emitting phosphor is a barium magnesium aluminate:Eu and/or strontium calcium barium phosphate:Eu and/or strontium aluminate:Eu and/or strontium chlorophosphate:Eu.

6. Low-pressure gas-discharge lamp according to Claim 4 and/or 5, **characterised in that** the proportion of blue-emitting phosphor in the mixture is between 0% and 10%.

7. Low-pressure gas-discharge lamp according to Claim 1, **characterised in that** the discharge tube is shaped by folding, deformation, welding or joining, in which the outer dimensions of the discharge vessel are only a fraction of the extended length of the discharge vessel.

8. Low-pressure gas-discharge lamp according to Claim 1 and/or 7, **characterised in that** the discharge vessel is in the shape of a double helix or is made from U shapes connected together and from short tube sections joined together via connecting channels.

## Revendications

1. Lampe de décharge de gaz basse pression pour influencer l'équilibre de mélatonine endogène, constituée d'un tube de décharge fabriqué en verre qui est en multicouche avec du phosphore sur l'intérieur et qui a aux deux extrémités du tube de décharge des électrodes pour l'application d'énergie et qui est rempli d'un gaz noble ou d'un mélange de gaz nobles avec du mercure, **caractérisée en ce que** les couches de phosphore sont appliquées l'une après l'autre, dans lequel la première couche de phosphore (2) située sur la surface de verre (1) est constituée d'un orthosilicate de métal alcalinoterreux activé par Eu ayant la formule (Ba,Sr,Ca)₂SiO₄:Eu (BOSE) ou d'un oxyorthosilicate de métal alcalinoterreux activé par Eu ayant la formule (Ba,Sr,Ca)₃SiO₅:Eu (SOOS) ou d'un aluminate choisi parmi le groupe des grenats ayant la formule Y₃Al₅O₁₂:Ce (YAG), lequel est excité à la fois par le rayonnement de mercure ultraviolet entre 180 nm et 400 nm et également par le rayonnement visible entre 400 nm et 550 nm émis par la décharge de mercure et la seconde couche de phosphore ou les couches de phosphore suivantes (3), dans lequel la lumière visible ayant un maximum d'émission dans la plage entre 500 nm et 650 nm est générée.

2. Lampe de décharge de gaz basse pression selon la revendication 1, **caractérisée en ce que** l'épaisseur de couche de la première couche de phosphore qui sert de couche de base est établie entre 0,2 mg/cm² et 2,0 mg/cm², de préférence 1.25 mg/cm², et l'épaisseur de couche de la seconde couche de phosphore qui sert de couche supérieure est établie entre 1,0 mg/cm² et 6.0 mg/cm², de préférence 3.25 mg/cm².

3. Lampe de décharge de gaz basse pression selon la revendication 1 et/ou 2, **caractérisée en ce que** le phosphore dans la première couche de phosphore a un maximum d'émission dans la région spectrale entre 560 nm et 620 nm.

4. Lampe de décharge de gaz basse pression selon une ou plusieurs des revendications 1 à 3, **caractérisée en ce que** la seconde couche de phosphore est constituée d'un ou plusieurs phosphores d'émission bande étroite qui émettent dans le bleu turquoise à un maximum d'émission entre 440 nm et 500 nm et/ou dans le vert à un maximum d'émission entre 535 nm et 555 nm et/ou dans le rouge à un maximum d'émission entre 605 nm et 630 nm.

5. Lampe de décharge de gaz basse pression selon la revendication 4, **caractérisée en ce que** le phosphore émettant dans le rouge est un oxyde d'yttrium:Eu et/ou un vanadate d'yttrium:Eu et/ou un fluorogermanate de magnésium/Mn, le phosphore émettant dans le vert est un phosphate de lanthanum:Ce,Tb et/ou un aluminate de magnésium:Ce,Tb, et le phosphore émettant dans le bleu turquoise est un aluminate de barium-magnésium:Eu et/ou un aluminate de strontium/Eu et/ou un chlorophosphate de strontium:Eu.

6. Lampe de décharge de gaz basse pression selon la revendication 4 et/ou 5, **caractérisée en ce que** la proportion de phosphore émettant dans le bleu dans le mélange est entre 0% et 10%.

7. Lampe de décharge de gaz basse pression selon la revendication 1, **caractérisée en ce que** le tube de décharge est conformé par pliage, déformation, soudage ou jonction, dans lequel les dimensions externes de l'ampoule de décharge sont seulement une fraction de la longueur étendue de l'ampoule de décharge.

8. Lampe de décharge de gaz basse pression selon la revendication 1 et/ou 7, **caractérisée en ce que** l'ampoule de décharge est selon la forme d'une double hélice ou est faite à partir de formes en U connectées ensemble et à partir de sections de tube courtes jointes ensemble via des canaux de connexion.
